# EUROPÄISCHE PATENTSCHRIFT

(11) **EP 1 000 942 B1**
(45) Veröffentlichungstag und Bekanntmachung des Hinweises auf die Patenterteilung: **13.02.2002**
(21) Anmeldenummer: 99122250.6
(22) Anmeldetag: 05.11.1999
(51) Int. Cl.: C07D 323/06

(54) **Trennung von flüssigen Gemischen enthaltend Formaldehyd, Trioxan, Alkohol und Hemiformal**
Separation of liquid mixtures containing formaldehyde, trioxane and hemiformal
Séparation de mélanges liquides contenant du formaldéhyde,du trioxanne et de l' hémiformal

(30) Priorität: 09.11.1998 DE 19851481
(43) Veröffentlichungstag der Anmeldung: 17.05.2000
(73) Patentinhaber: Ticona GmbH, 65451 Kelsterbach (DE)
(72) Erfinder: Reichl, Albert Dr., 65812 Bad Soden (DE); Kleiber, Michael, Dr., 65929 Frankfurt (DE); Rosenberg, Michael, 65527 Niedernhausen (DE); Scheid, Dirk, 65529 Waldems (DE); Sommerfeld, Dieter, 65510 Hünstetten-Beuerbach (DE); Nickelfeld, Wolfgang, 65933 Frankfurt (DE)

(56) Entgegenhaltungen:
- EP-A- 0 133 669
- EP-A- 0 754 689
- WO-A-96/22986

## Beschreibung

Die Erfindung betrifft ein Verfahren zur Trennung von flüssigen Gemischen enthaltend Formaldehyd, Trioxan, Alkohol, aus dem Formaldehyd und dem Alkohol gebildetes Hemiformal, übliche Nebenkomponenten und bis zu 5 Gew.-% Wasser. Dabei wird das flüssige Gemisch bei Unterdruck, Normaldruck oder Überdruck in geeigneten Apparaten, die in entsprechender Weise miteinander zu verschalten sind, diskontinuierlich oder kontinuierlich destilliert und das Trioxan in sehr hoher Reinheit gewonnen. Die anderen im Gemisch enthaltenen Wertstoffe Formaldehyd und Alkohol können wahlweise ebenfalls abgetrennt und rückgeführt oder anderweitig verwendet werden, während die Nebenkomponenten ausgeschleust werden.

Zur Herstellung von technischen Kunststoffen insbesondere von Polyacetalen wie Polyoxymethylen wird hochreines Trioxan benötigt. Die Qualität des Kunststoffes, d.h. der erreichbare Polymerisationsgrad, hängt dabei neben den Polymerisationsbedingungen vorrangig von der Reinheit des Trioxans ab.

Es sind verschiedene Verfahren zur Herstellung von Trioxan bekannt, z.B. homogen oder heterogen katalysiert aus wäßrigen Formaldehydlösungen (AT-252913) oder heterogen katalysiert aus gasförmigem Formaldehyd an Heteropolysäuren (EP-606056). Unabhängig vom Herstellverfahren fällt Trioxan jedoch niemals als reiner Stoff sondern immer im Gemisch mit nicht umgesetztem Formaldehyd, Wasser und geringen Mengen anderer Komponenten wie Methanol, Methylformiat, Methylal, Ameisensäure, Dioxolan und Tetroxan an, die allgemein als Nebenkomponenten bezeichnet werden. Zur weiteren Verwendung des Trioxans z.B. für die Polymerisation muß dieses insbesondere vom Formaldehyd abgetrennt werden und darf nur geringe Mengen an Nebenkomponenten enthalten.

Allgemein geläufig ist die Trennung von Trioxan aus wäßrigen Trioxan-Formaldehyd-Gemischen, die mehr als 10 Gew.-% Wasser enthalten. Bekannt ist aber auch die Trennung gasförmiger Gemische aus Formaldehyd und Trioxan.

Die Trennung wäßriger Gemische erfolgt bisher insbesondere über die azeotrope Destillation (AT-252913 und JP 83-171278). Bei diesem Verfahren kann nicht umgesetzter Formaldehyd wieder in den Reaktor zurückgeführt und dort weiter zu Trioxan umgesetzt werden. Ein Grenzwert für die erreichbare Reinheit des somit gewonnen Trioxans ergibt sich allerdings durch das bei 92°C und 1 bar siedende Azeotrop von Trioxan und Wasser, welches ca. 70 Gew.-% Trioxan enthält. Als Nachteil dieser Verfahren ist die Gefahr der Feststoffbildung durch Polymerisation von Formaldehyd oder Bildung von para-Formaldehyd vor allem im Bereich des Kolonnenkopfes bekannt. Zur Vermeidung derselben müssen alle Apparateteile entweder auf Temperaturen >100 °C (bei 1 bar Formaldehyd-Partialdruck) aufgeheizt oder mit einer Flüssigkeit benetzt werden.

Ein weiteres Verfahren zur Trennung von Formaldehyd und Trioxan aus wäßrigen Lösungen besteht in der Extraktion des Trioxans mit organischen Lösungsmitteln, in denen Trioxan eine höhere physikalische Löslichkeit besitzt als der Formaldehyd. Dieses Verfahren wird ausschließlich zur Abtrennung des Trioxans aus der wäßrigen Phase eingesetzt. Als organische Extraktionsmittel werden beispielsweise gesättigte aliphatische oder aromatische Kohlenwasserstoffe oder Halogenkohlenwasserstoffe (EP-583907) verwendet, die wenig oder gar nicht mit Wasser mischbar sind. Ein Nachteil der Extraktion ist, daß mit dem organischen Lösungsmittel ein zusätzlicher Hilfsstoff in das Verfahren eingebracht wird und eine anschließende Aufarbeitung auch der organischen Phase erforderlich ist. Ein weiterer Nachteil besteht darin, daß mitunter auch beträchtliche Teile des Trioxans in der wäßrigen Phase verbleiben. Große Mengen an Trioxan müssen daher im Kreis geführt werden oder gehen beim Aufarbeitungsverfahren verloren.

Als weitere Möglichkeit der selektiven Trennung aus einer wäßrigen Phase wird die Kristallisation des Trioxans aus wäßrigen Formaldehyd-Trioxan-Gemischen beschrieben (DE-3508668). Die Trioxankonzentration in dem wäßrigen Gemisch muß dabei mehr als 50 Gew.-% betragen.

Bei der Herstellung von Trioxan durch Trimerisierung von Formaldehyd aus wäßrigen Formalinlösungen werden in der Regel die oben genannten Verfahren der azeotropen Destillation, Extraktion und gegebenenfalls der Kristallisation in geeigneter Weise miteinander verknüpft, um Trioxan in der geforderten hohen Reinheit zu erhalten.

Für die Trennung von gasförmigen Gemischen von Formaldehyd und Trioxan sind Verfahren der selektiven Absorption bekannt. Dabei wird entweder der Formaldehyd chemisorbiert und das Trioxan in der Gasphase belassen (GB-1245990) oder es erfolgt umgekehrt eine selektive Physisorption des Trioxans (EP-A-680959). Da keine flüssige Phase gefunden wurde, in der entweder nur der Formaldehyd oder nur das Trioxan löslich ist, werden bei der Absorption auch Anteile der jeweils anderen Spezies gebunden. Daher entsteht bei der selektiven Absorption von Formaldehyd ein beträchtlicher Verlust am Wertstoff Trioxan, während bei der selektiven Absorption von Trioxan auch mit diesem Verfahren die für die Polymerisation nötige Reinheit von Trioxan nicht erzielt werden kann.

Ein Verfahren zur Trennung eines gasförmig und wasserarm vorliegenden Gemisches aus Formaldehyd und Trioxan wird in der noch nicht veröffentlichten deutschen Patentanmeldung Nr. 198 336 20.9 beschrieben. Dieses Verfahren beinhaltet die Absorption in einem Alkohol und eine nachgeschaltete Abtrennung des Trioxans vom Alkohol bzw. vom Hemiformal durch Kristallisation. Das Verfahren bildet einen möglichen Schritt in einem neuen Prozeß zur Herstellung von Trioxan aus Methanol, bestehend aus den Schritten nicht oxidative Dehydrierung (DE-3920811), Formaldehydabtrennung (deutsche Patentanmeldungen Nr. 197 476 47.3 und Nr. 197 483 80.1), Formaldehydtrimerisierung (EP-A-606056, EP-A-691388) und Trioxanabtrennung. Allerdings weist auch das nach diesem Verfahren gewonnene Trioxan noch nicht die für eine Polymerisation notwendige Reinheit auf.

Die EP-A- 754 689 beschreibt die Abtrennung von Trioxan aus einem wässrigen Gemisch durch Destillation. Hier enthält das Gemisch lediglich Trioxan, Formaldehyd und Wasser.

Es bestand nun die Aufgabe, ein Verfahren zu entwickeln, nach dem Trioxan in hoher, insbesondere für die Polymerisation geeigneter Reinheit gewonnen werden kann. Dabei sollte insbesondere das Problem der Abtrennung von Trioxan aus einem flüssigen Trioxan-Formaldehyd-Gemisch gelöst werden. Die anderen im Gemisch enthaltenen Wertstoffe Formaldehyd und Alkohol sollten mit diesem Verfahren möglichst ebenfalls abgetrennt und rückgeführt oder anderweitig verwendet werden können, während die teilweise für die Polymerisation schädlichen Nebenkomponenten ausgeschleust werden sollten.

Diese Aufgabe wird erfindungsgemäß gelöst, indem ein flüssiges Gemisch aus Formaldehyd, Trioxan, einem Alkohol, entsprechenden Hemiformalen, geringen Mengen an leichter- und schwerersiedenden Nebenkomponenten und maximal 5 Gew.-% Wasser in geeigneter Weise in einem oder mehreren, entsprechend verschalteten Apparaten bei Unterdruck, Normaldruck oder Überdruck destilliert und somit das Trioxan in hoch reiner Form gewonnen wird, wobei es je nach Verfahrensführung als Destillat, Sumpfprodukt oder Seitenabzug anfallen kann.

Die Erfindung betrifft daher ein Verfahren zur Gewinnung von Trioxan, wobei aus einem flüssigen Gemisch enthaltend Trioxan, Formaldehyd, Alkohol, aus dem Formaldehyd und dem Alkohol gebildete Hemiformale und maximal 5 Gew.-% Wasser das Trioxan durch Destillation abgetrennt und in hoch reiner Form gewonnen wird.

Die Erfindung betrifft auch die Verwendung des somit gewonnenen hoch reinen Trioxans zur Herstellung von Polymeren und Brennstoffen bzw. zur Gewinnung von Formaldehyd durch Depolymerisation des Trioxans.

Wesentlich für das erfindungsgemäße Verfahren ist, daß das zu trennende flüssige Gemisch wasserarm oder wasserfrei ist, d.h. es enthält im allgemeinen maximal 5 Gew.-%, vorzugsweise maximal 3 Gew.-% Wasser.

Das erfindungsgemäß gewonnene hoch reine Trioxan weist eine Reinheit von mindestens 95 Gew.-%, vorzugsweise mindestens 97 Gew.-% und besonders bevorzugt mindestens 99 Gew.-% Trioxan auf. Vorteilhaft enthält dazu das zu trennende flüssige Gemisch einen Gehalt an Trioxan im Bereich von 70 bis 95 Gew.- %, besonders vorteilhaft im Bereich von 83 bis 88 Gew.-%.

Bei dem im flüssigen Gemisch enthaltenen Alkohol, der mit dem Formaldehyd teilweise Hemiformale bilden kann, handelt es sich vorzugsweise um einen einwertigen Alkohol, z.B. Cyclohexanol, Methanol, Propanol oder Butanol. Es ist aber auch der Einsatz anderer, auch mehrwertiger Alkohole wie Glyzerol, di-Ethylen-Glykol, tri-Ethylen-Glykol, tri-Ethanolamin, Butantriol oder Pentantriol möglich. Es ist auch möglich, ein Gemisch verschiedener Alkohole zu verwenden. Vorteilhafter Weise sollte der Alkohol jedoch einen höheren Siedepunkt als Trioxan aufweisen.

Das flüssige Gemisch kann auch übliche bei der Herstellung von Trioxan anfallende Nebenkomponenten wie Methanol, Methylformiat, Tetroxan, Dioxolan, Trioxyether und Spuren von Ameisensäure enthalten. Der Anteil der Nebenkomponenten im flüssigen Gemisch beträgt dabei im allgemeinen 1 bis 10 Gew.-%, vorzugsweise 2 bis 5 Gew.-%. Die Nebenkomponenten können durch geeignete Verfahrensführung in separaten Produktströmen wahlweise ebenfalls abgetrennt und als flüssige oder gasförmige Produktströme gewonnen werden. Dies kann insbesondere durch Destillation in mehreren miteinander verschalteten Destillierapparaten erreicht werden.

Auch die im flüssigen Gemisch enthaltenen Wertstoffe Formaldehyd und Alkohol können vorteilhaft durch geeignete Verschaltung von Destillierapparaten simultan in separaten Produktströmen gewonnen und in den Prozeß rückgeführt oder anderweitig verwendet werden.

Das erfindungsgemäße Verfahren kann in einem einzigen oder in mehreren miteinander verschalteten Destillierapparaten durchgeführt werden. Diese können diskontinuierlich oder kontinuierlich betrieben werden, wobei die kontinuierliche Betriebsweise bevorzugt ist. Vorteilhaft, insbesondere bei Verwendung nur eines Destillierapparates, weisen die Destillierapparate mehrere Kühl- und Kondensationszonen mit unterschiedlicher Temperatur auf. Dadurch sowie durch die Verwendung mehrerer miteinander verschalteter Destillierapparate kann eine hohe Reinheit des Trioxans und die gleichzeitige Gewinnung von Formaldehyd und Alkohol sowie der Nebenkomponenten erreicht werden. Abhängig von der Verfahrensführung und der Verschaltung der Destillierapparate fallen die verschiedenen Produktströme wahlweise flüssig oder gasförmig als Destillat, Sumpfprodukt oder Seitenabzug an.

Überraschend zeigt sich, daß nach dem erfindungsgemäßen Verfahren, im Gegensatz zu den bekannten Verfahren zur destillativen Abtrennung von Trioxan, keine Feststoffbildung in den Destillierapparaten auftritt. Anscheinend wird durch das gleichzeitige Vorhandensein von Trioxan in der Dampfphase die Bildung von festem para-Formaldehyd in den Destillationsapparaten wirkungsvoll unterdrückt.

Ein besonderer Vorteil des erfindungsgemäßen Verfahrens besteht darin, daß bei entsprechender Prozeßführung der in der Zuspeisung enthaltene Formaldehyd gasförmig gewonnen werden und daher z.B. in den eingangs genannten Prozeßschritt Formaldehydtrimerisierung rückgeführt werden kann.

Vorteilhaft ist das erfindungsgemäße Verfahren auch geeignet, Trioxan aus einem flüssigen und wasserarm vorliegenden Gemisch abzutrennen, wie es in der Absorptions- oder der Kristallisationsstufe nach dem in der deutschen Anmeldung Nr. 198 336 20.9 beschriebenen Verfahren und damit in dem o.g. neuen Prozeß zur Herstellung von Trioxan aus Methanol anfällt.

Das nach dem erfindungsgemäßen Verfahren hergestellte Trioxan eignet sich für alle bekannten Einsatzgebiete wie die Polymerisation zu Kunststoffen (z.B. zu Polyacetalen, insbesondere Polyoxymethylen), die Herstellung von Brennstoffen und die Depolymerisation zu Formaldehyd, was die Weiterverwendung für alle Formaldehyd-Reaktionen erlaubt, insbesondere wenn dafür sehr reiner Formaldehyd erforderlich ist.

Im Unterschied zum Verfahren nach AT-252913 oder JP 83-171278 wird die destillative Trennung beim erfindungsgemäßen Verfahren aus einem wasserarmen flüssigen Gemisch ohne Einsatz zusätzlicher stofflicher Hilfsmittel durchgeführt. D.h. das flüssige Gemisch enthält im wesentlichen Trioxan, Formaldehyd, Alkohol, entsprechende Hemiformale, übliche bei der Reaktion entstehende Nebenkomponenten in geringen Anteilen und 0 bis 5 Gew.-% Wasser. Im Laufe des Verfahrens werden auch keine weiteren Stoffe oder Hilfsstoffe zugeführt. Da der Vorteil des neuen Prozesses insgesamt die wasserarme Herstellung von Trioxan ist, stellen die Verfahren nach AT-252913 und JP 83-171278 hier keine brauchbaren Lösungen dar.

Das erfindungsgemäße Verfahren zeichnet sich gegenüber den bisher eingesetzten Verfahren durch folgende Vorteile aus:
- sehr hohe Reinheit und Ausbeute an Trioxan,
- Wertstoffe Formaldehyd, Alkohol sowie Hemiformal sind in separaten, aus dem Verfahren abgezogenen Strömen erhältlich und können rückgeführt oder anderweitigen Verwendungen zugeführt werden,
- Ausschleusung von leicht- und schwersiedenden Verunreinigungen,
- keine stofflichen Hilfsmittel erforderlich,
- energetisch vorteilhaft durch interne Energierückgewinnung,
- Vermeidung der Feststoffbildung (para-Formaldehyd) durch gleichzeitige Anwesenheit von Trioxan in der Gasphase bzw. von Trioxan, Alkohol und Hemiformal in der Flüssigphase.

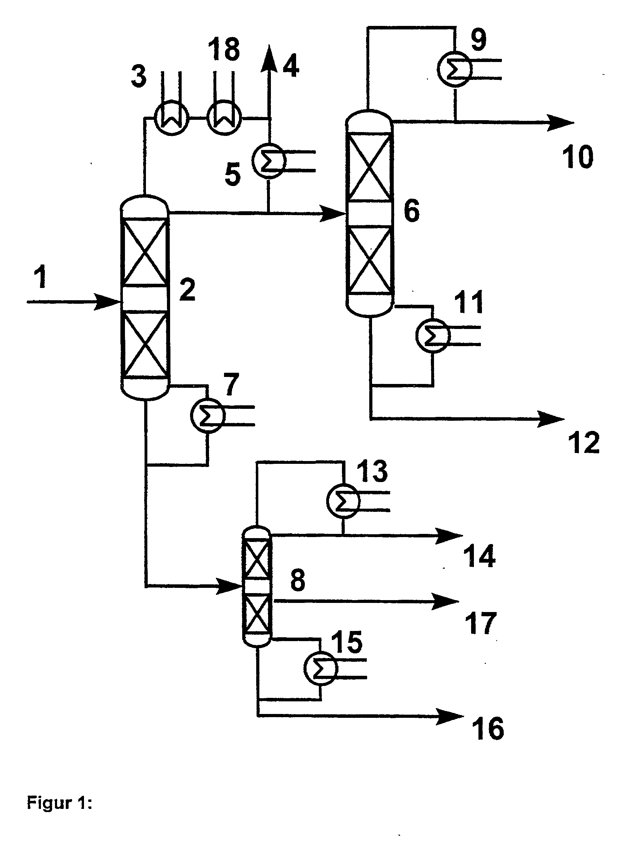

In Figur 1 ist eine mögliche apparative Umsetzung und Apparateverschaltung des erfindungsgemäßen Verfahrens dargestellt: Das flüssige Ausgangsgemisch 1 wird einem Destillierapparat 2 zugeführt, der hier als Kolonne ausgeführt ist. Am Kopf der Kolonne 2 wird Formaldehyd zusammen mit etwas Trioxan nach den Teilkondensatoren 3 und 18 als gasförmiger Strom 4 abgezogen. Der übrige Brüdenstrom, vorwiegend Trioxan sowie Leichtersieder enthaltend, wird mit dem Wärmetauscher 5 im flüssigen Zustand gehalten, teilweise auf die Kolonne 2 zurückgeführt und teilweise einer zweiten Kolonne 6 zugeführt. Die im Sumpf der Kolonne 2 vorliegende Flüssigkeit enthält vorwiegend Alkohol, Hemiformal, höhersiedende Nebenkomponenten und Trioxan. Ein Teil davon wird im Verdampfer 7 verdampft und auf die Kolonne 2 zurückgeführt. Ein anderer Teilstrom wird einer weiteren Kolonne 8 zugeführt.

In der Kolonne 6 fallen als Kopfprodukt nach Kondensation im Kondensator 9 die leichtersiedenden Nebenkomponenten, beispielsweise Methanol und Wasser, aber auch Trioxan (welches mit Wasser ein Azeotrop bildet) flüssig an. Das Kondensat wird teilweise auf die Kolonne 6 zurückgeführt und teilweise als Strom 10 abgezogen. Die im Sumpf der Kolonne 6 vorliegende Flüssigkeit enthält Trioxan sehr hoher Reinheit. Ein Teil davon wird im Verdampfer 11 verdampft und auf die Kolonne 6 rückgeführt. Ein anderer Teilstrom wird als Strom 12 abgezogen.

In der Kolonne 8 fallen als Kopfprodukt nach Kondensation im Kondensator 13 vorwiegend die zwischen dem Trioxan und dem Alkohol siedenden

Nebenkomponenten flüssig an. Das Kondensat wird teilweise auf die Kolonne 8 zurückgeführt und teilweise als Strom 14 abgezogen. Die im Sumpf der Kolonne 8 vorliegende Flüssigkeit enthält die höhersiedenden Nebenkomponenten. Ein Teil davon wird im Verdampfer 15 verdampft und auf die Kolonne 8 rückgeführt. Ein anderer Teilstrom wird als Strom 16 abgezogen. Der Alkohol wird mit einem flüssigen oder dampfförmigen Seitenabzug 17 aus der Kolonne 8 entnommen.

Die Kolonne 2 wird bei Unterdruck, Normaldruck oder Überdruck (vorzugsweise Normaldruck) betrieben. Die Temperaturen im Sumpf der Kolonne 2 liegen zwischen 80 und 200°C, vorzugsweise zwischen 110 und 150°C. Unter den letztgenannten Bedingungen wird im Sumpf der Kolonne 2 der Formaldehyd aus den Hemiformalen teilweise abgespalten. Die Temperaturen im Teilkondensator 3 liegen zwischen 50 und 140°C, vorzugsweise zwischen 70 und 100°C. Im Teilkondensator 18 werden Temperaturen zwischen 40 und 120°C, vorzugsweise zwischen 60 und 80°C eingestellt. Die Temperaturen im Wärmetauscher 5 liegen zwischen 50 und 140°C, vorzugsweise zwischen 70 und 100°C.

Im beschriebenen Verfahren führt die Temperaturführung der Teilkondensatoren und Wärmetauscher am Kopf der Kolonne 2 dazu, daß der im Sumpf abgespaltene Formaldehyd am Kopf gasförmig zusammen mit etwas Trioxan abgezogen werden kann, wobei durch die gleichzeitige Anwesenheit von Trioxan die bei anderen Prozessen mit Formaldehyd vielfach problematische Feststoffbildung (para-Formaldehyd) vermieden wird. Über eine unterschiedliche Temperaturführung der Teilkondensatoren und Wärmetauscher lassen sich die Menge und die Zusammensetzung des gasförmigen Abzuges sowie der Formaldehydanteil im Kopfprodukt der Kolonne 2 steuern.

Die Kolonne 6 wird ebenfalls bei Unterdruck, Normaldruck oder Überdruck (vorzugsweise Normaldruck) betrieben. Die Temperaturen im Sumpf der Kolonne 8 liegen zwischen 80 und 150°C, vorzugsweise zwischen 100 und 130°C. Im Kopf der Kolonne 6 treten Temperaturen zwischen 0 und 100°C, vorzugsweise zwischen 30 und 70°C auf.

Auch die Kolonne 8 wird bei Unterdruck, Normaldruck oder Überdruck (vorzugsweise Normaldruck) betrieben. Die Temperaturen im Sumpf der Kolonne 8 liegen zwischen 100 und 250°C, vorzugsweise zwischen 150 und 180°C. Im Kopf der Kolonne 8 treten Temperaturen zwischen 80 und 180°C, vorzugsweise zwischen 100 und 140°C auf. Die dampfförmige oder flüssige Seitenstromentnahme erfolgt entspechend bei Temperaturen zwischen 80 und 250°C, vorzugsweise zwischen 130 und 180°C.

Die oben beschriebene Apparateverschaltung nach Figur 1 stellt nur eine denkbare Variante des erfindungsgemäßen Verfahrens dar. Insbesondere ist es auch möglich, zuerst die Trennung des Trioxans von den Leichtersiedern, und danach die Trennung von den Schwerersiedern vorzunehmen.

Durch eine Variation der Anzahl und Anordnung der Seitenabzügen gelangt man im erfindungsgemäßen Verfahren ebenfalls zu unterschiedlichen Anordnungen von Kolonnen.

Eine weitere Variante des erfindungsgemäßen Verfahrens ist die teilweise Rückführung der aus einer Kolonne erhaltenen Produktströme zur gezielten Veränderung der Zusammensetzung der Zuspeisung zu dieser Kolonne. So läßt sich z.B. ein Teilstrom aus dem am Kopf der Kolonne 2 gewonnenen Destillats entnehmen und der Zuspeisung 1 beimischen. Auf diese Weise wird der Anteil z.B. des Trioxans in der Zuspeisung 1 verändert, was wiederum Auswirkungen auf die Zusammensetzung der Ströme in der Kolonne und der Produktströme aus der Kolonne 2 hat. Schwankungen in der Zusammensetzung der Zuspeisung 1, die durch einen früheren Prozeßschritt bedingt sind und sich nachteilig auf die Produkte der Destillation auswirken, lassen sich auf diese Weise ebenfalls ausgleichen.

Im Gegensatz zur in Figur 1 dargestellten kontinuierlichen Verfahrensführung kann die destillative Aufarbeitung im erfindungsgemäßen Verfahren auch diskontinuierlich erfolgen. Vorteilhaft ist hierbei die mehrfache Nutzung einzelner Kolonnen für verschiedene Trennschritte. Nachteilig sind die geringeren Durchsätze und damit längeren Produktionszeiten - gleiche Kolonnengrößen vorausgesetzt.

Als eine Ergänzung zur in Figur 1 gezeigten Anordnung läßt sich im erfindungsgemäßen Verfahren eine zusätzlich Aufarbeitung des aus der Kolonne 6 austretenden Stromes 10 durchführen. Insbesondere ist Methanol, aber auch Trioxan abtrennbar, letzteres z.B. durch eine Destillation bei verändertem Druck zur Überwindung des Azeotrops mit Wasser.
Wird im Gegensatz zu der in Figur 1 gezeigten Anordnung im erfindungsgemäßen Verfahren ein Alkohol zur Hemiformalbildung verwendet, der einen niedrigeren Siedepunkt als Trioxan aufweist, so ergeben sich ebenfalls andere Trennschritte. Beispielsweise können in diesem Fall in einer ersten Kolonne Trioxan und Schwerersieder von Alkohol und Leichtersiedern getrennt werden. In zwei weiteren Kolonnen werden anschießend Trioxan und der Alkohol jeweils abgetrennt.

Die destillative Trennung läßt sich im erfindungsgemäßen Verfahren bei Unter-Normal- oder Überdruck betreiben. Bei Unterdruckfahrweise erfolgt die Destillation schonender, jedoch bei verminderten Durchsätzen, Problemen durch Leckagen und der Notwendigkeit der Vakuumerzeugung. Bei Überdruckfahrweise sind die Durchsätze größer, und die Neigung des Formaldehyds zur Feststoffbildung ist wegen der höheren Temperaturen geringer. Nachteilig sind die stärkere Bildung von Nebenkomponenten, die höhere Festigkeit der Apparate, Probleme mit Leckagen und die Notwendigkeit der Druckerzeugung.

Als Destillierapparate werden bevorzugt Kolonnen, insbesondere Oberflächenrektifikatoren, wie gepackte Kolonnen oder Bodenkolonnen eingesetzt, wobei Bodenkolonnen ein großes Rückhaltevermögen und gepackte Kolonnen eine große spezifische Oberfläche aufweisen. Besonders bevorzugte Kolonnen sind Glockenbodenkolonnen, Rieselkolonnen, Füllkörperkolonnen und Kolonnen mit geordneten Packungen. Um eine möglichst niedrige Kolonnenhöhe bei hohen Durchsätzen zu erreichen, werden bevorzugt Kolonnen mit solchen Einbauten eingesetzt, die eine möglichst große Austauschfläche bereitstellen, z.B. mit strukturierten Packungen.

Im erfindungsgemäßen Verfahren läßt sich eine vorteilhafte, mehrfache Nutzung der Energien durch interne Energierückgewinnung durchführen. So läßt sich beispielsweise in der in Figur 1 gezeigten Verschaltung, bei Prozeßführung mit genügend unterschiedlichen Temperatumiveaus, die Abwärme von Verdampfer 15 für die Beheizung von Verdampfer 7 oder 11, anschließend für die Temperierung der Teilkondensatoren 3 und 18 sowie des Wärmetauschers 5 und weiter für die Temperierung des Kondensators 9 verwenden. Eine weitere Möglichkeit besteht darin, die am Kondensator 13 abgeführte Energie zur Beheizung des Verdampfers 11 zu nutzen.

Das erfindungsgemäße Verfahren sei nachfolgend anhand einiger experimenteller Untersuchungen illustriert. Es wurde dabei die Kolonne 2 bzw. die Kolonne 6 aus Figur 1 im Labor nachgestellt und jeweils einzeln kontinuierlich bei Normaldruck betrieben. Die Heizleistung in den Verdampfern 7 bzw. 11 wurde so eingestellt, daß der Flüssigkeitsstand im Sumpf konstant blieb und somit die Massenbilanz um die Kolonne jeweils erfüllt war. Von sämtlichen Flüssigkeitsströmen wurden Proben entnommen, welche mit einem Gaschromatographen analysiert wurden.

Aufbau der Kolonne 2 bei den Laborversuchen:
- Schüsse:: aus Glas DN50, bestückt im Verstärkungs- und Abtriebsteil mit je 1 m strukturierter Packung CY der Firma Sulzer
- Kopf:: Bauart Kaminsky mit drei unterschiedlich temperierbaren Wärmetauschern (vgl. Figur 1)
- Feed:: flüssiges Gemisch enthaltend u.a. Trioxan, Cyclohexanol, Formaldehyd, Methanol und Wasser. Formaldehyd darin überwiegend gebunden in Form von Cyclohexyl-Hemiformal. Zuspeisung in die Kolonnenmitte mit Waage/Pumpe/Dosierregler-Einheit
- Sumpfentnahme:: mit Waage/Pumpe/Dosierregler-Einheit
- Produktströme am Kolonnenkopf:: a) gasförmig
b) flüssig (Destillat)
Aufbau der Kolonne 6 bei den Laborversuchen:
- Schüsse:: aus Glas DN50, bestückt im Verstärkungs- und Abtriebsteil mit je 1 m strukturierter Packung BX der Firma Sulzer
- Kopf:: Bauart Kaminsky
- Feed:: flüssiges Gemisch enthaltend u.a. Trioxan, Formaldehyd, Methanol und Wasser.
Zuspeisung in die Kolonnenmitte mit Waage/Pumpe/Dosierregler-Einheit
- Sumpfentnahme:: mit Waage/Pumpe/Dosierregler-Einheit
- Produktstrom am Kolonnenkopf:: flüssig (Destillat)

Bei der Versuchsbeschreibung werden im weiteren die folgenden Abkürzungen benutzt:
- FA: Formaldehyd
- MeOH: Methanol
- H2O: Wasser
- AS: Ameisensäure
- TOX: Trioxan
- CHOL: Cyclohexanol
- n.b.: nicht bestimmt

Alle Prozentangaben sind als Massenprozente zu verstehen.

### Beispiel 1, Kolonne 2 - Versuch HETO4

Als Verdampfer 7 wurde ein Naturumlaufverdampfer eingesetzt, in welchem ein Holdup von ca. 700 cm³ eingestellt wurde. Die Teilkondensatoren 3 und 18 wurden bei 50°C betrieben, der Wärmetauscher 5 hingegen bei 70°C. Die Versuchsparameter und -ergebnisse sind in Tabelle 1 zusammengestellt.

**Tabelle 1**

| Versuchsparameter und -ergebnisse beim Versuch HETO4 | | | | | | | | | |
|---|---|---|---|---|---|---|---|---|---|
| T_{Sumpf} °C | T_{Kopf} °C | Rücklaufverh. g/g | Bezeichnung Fraktion | Massenstrom g/h | FA % | MeOH % | H2O % | TOX % | CHOL % |
| 117 | 110 | 2:1 | Zuspeisung | 750 | 4,5 | 1,2 | 0,3 | 86,7 | 7,3 |
| | | | Sumpf | 102 | 0,2 | <0,1 | 0,05 | 75,7 | 24,0 |
| | | | Destillat | 602 | 2,4 | 1,4 | 0,4 | 95,7 | <0,1 |
| | | | gasf. Abzug | | ca. 45 | n.b. | n.b. | ca. 55 | n.b. |

Das Hemiformal wird nahezu vollständig gespalten, so daß das Sumpfprodukt nur noch 0,2 % Formaldehyd aufweist. Das Destillat ist nahezu frei von Cyclohexanol und enthält neben den Leichtersiedem Methanol und Wasser noch 2,4 % Formaldehyd.

### Beispiel 2, Kolonne 2 - Versuch HETO5a

Als Verdampfer 7 wurde ein Fallfilmverdampfer eingesetzt, in welchem ein Holdup von ca. 400 cm³ eingestellt wurde. Der Teilkondensator 3 wurde bei 70°C betrieben, der Teilkondensator 18 bei 50°C, der Wärmetauscher 5 bei 70°C. Die Zusammensetzung des gasförmigen Abzuges wurde per Online-GC gemessen. Die Versuchsparameter und -ergebnisse sind in Tabelle 2 zusammengestellt.

**Tabelle 2**

| Versuchsparameter und -ergebnisse beim Versuch HETO5a | | | | | | | | | |
|---|---|---|---|---|---|---|---|---|---|
| T_{Sumpf} °C | T_{Kopf} °C | Rücklaufverh. g/g | Bezeichnung Fraktion | Massenstrom g/h | FA % | MeOH % | H2O % | TOX % | CHOL % |
| 119 | 109 | 2:1 | Zuspeisung | 750 | 6,1 | 1,0 | 0,3 | 87,0 | 5,6 |
| | | | Sumpf | 60 | 0,2 | <0,1 | <0,05 | 76,7 | 23,0 |
| | | | Destillat | 626 | 3,5 | 1,1 | 0,3 | 95,1 | <0,005 |
| | | | gasf. Abzug | | 64,2 | 0,6 | 0,53 | 34,7 | <0,1 |

Auch bei Einsatz eines Verdampfers mit einem gegenüber Beispiel 1 kleineren Holdup und bei etwas höheren Hemiformal-Konzentrationen in der Zuspeisung wird Hemiformal im Sumpf nahezu vollständig gespalten. Bedingt durch die andere Temperierung der Wärmetauscher am Kolonnenkopf ist gegenüber Beispiel 1 die Formaldehyd-Konzentration im Destillat höher. Die Analytik des Cyclohexanols im Destillat erfolgte mit einer niedrigeren Nachweisgrenze des GC-Meßverfahrens als bei Beispiel 1. Es zeigte sich, daß im Destillat weniger als 50 ppm an Cyclohexanol enthalten sind. Die Verringerung der Sumpfentnahmemenge von ca. 100 g/h bei Beispiel 1 auf ca. 60 g/h bei Beispiel 2 hat keinen Einfluß auf die Zusammensetzungen der Produktströme.

### Beispiel 3, Kolonne 2 - Versuch HETO5b

Als Verdampfer 7 wurde wie in Beispiel 2 ein Fallfilmverdampfer eingesetzt, in welchem ein Holdup von ca. 400 cm³ eingestellt wurde. Anders als bei Beispiel 2 wurden der Teilkondensator 3 und der Wärmetauscher 5 bei 100°C betrieben, während am Teilkondensator 18 wieder 50°C eingestellt waren. Die Zusammensetzung des gasförmigen Abzuges wurde per Online-GC gemessen. Die Versuchsparameter und -ergebnisse sind in Tabelle 3 zusammengestellt.

**Tabelle 3**

| Versuchsparameter und -ergebnisse beim Versuch HETO5b | | | | | | | | | |
|---|---|---|---|---|---|---|---|---|---|
| T_{Sumpf} °C | T_{Kopf} °C | Rücklaufverh. g/g | Bezeichnung Fraktion | Massenstrom g/h | FA % | MeOH % | H2O % | TOX % | CHOL % |
| 122 | 109 | 2:1 | Zuspeisung | 750 | 6,1 | 1,0 | 0,3 | 87,0 | 5,6 |
| | | | Sumpf | 60 | 0,3 | <0,1 | <0,05 | 62,3 | 37,2 |
| | | | Destillat | 617 | 2,9 | 1,1 | 0,3 | 95,7 | <0,005 |
| | | | gasf. Abzug | | 57,3 | 0,6 | 0,43 | 41,7 | <0,1 |

Die Erhöhung der Temperatur im Teilkondensator 3 und Wärmetauscher 5 von 70°C in Beispiel 2 auf 100°C bewirkt eine Verminderung des Formaldehyd-Anteils im Destillat, welches wiederum weniger als 50 ppm an Cyclohexanol enthält.

### Beispiel 4, Kolonne 2 - Versuch HETO7

Als Verdampfer 7 wurde wie in Beispiel 2 und 3 ein Fallfilmverdampfer eingesetzt, in welchem diesmal jedoch ein Holdup von ca. 200 cm³ eingestellt wurde. Der Teilkondensator 3 wurde bei 95°C betrieben, der Teilkondensator 18 bei 60°C und der Wärmetauscher 5 bei 85°C. Die Zusammensetzung des gasförmigen Abzuges wurde per Online-GC gemessen. Die Versuchsparameter und -ergebnisse sind in Tabelle 4 zusammengestellt.

**Tabelle 4**

| Versuchsparameter und -ergebnisse beim Versuch HETO7 | | | | | | | | | | |
|---|---|---|---|---|---|---|---|---|---|---|
| T_{Sumpf} °C | T_{Kopf} °C | Rücklaufverh. g/g | Bezeichnung Fraktion | Massenstrom g/h | FA % | MeOH % | H2O % | AS % | TOX % | CHOL % |
| 125 | 108 | 2:1 | Zuspeisung | 750 | 4,8 | 1,1 | 0,3 | 0,073 | 86,9 | 6,9 |
| | | | Sumpf | 100 | 0,3 | <0,1 | <0,05 | 0,106 | 42,3 | 57,4 |
| | | | Destillat | 626 | 2,5 | 1,3 | 0,3 | 0,058 | 95,8 | <0,013 |
| | | | gasf. Abzug | | 53,7 | 0,85 | 0,46 | <0,1 | 45,0 | <0,1 |

Auch bei dem gegenüber Beispiel 3 um etwa die Hälfte verkleinerten Flüssigkeitsholdup im Verdampfer 7 wird das Hemiformal im Sumpf nahezu vollständig gespalten. Die Nebenkomponente Ameisensäure wird unter den Destillationsbedingungen nicht gebildet. Im Destillat sind weniger als 130 ppm an Cyclohexanol enthalten.

### Beispiel 5, Kolonne 6 - Versuch 980819-DST

Die Zuspeisung zu der Kolonne bei diesem Versuch entsprach etwa dem in der Kolonne 2 (vgl. Figur 1) am Kopf gewonnenen Strom, in den Beispielen 1 bis 4 als Destillat bezeichnet. Als Verdampfer 11 wurde ein Naturumlaufverdampfer eingesetzt, in welchem ein Holdup von ca. 700 cm³ eingestellt wurde. Die Temperierung des Kolonnenkopfes erfolgte bei 62°C. Die Versuchsparameter und - ergebnisse sind in Tabelle 5 zusammengestellt.

**Tabelle 5**

| Versuchsparameter und -ergebnisse beim Versuch 980819-DST | | | | | | | | |
|---|---|---|---|---|---|---|---|---|
| T_{Sumpf} °C | T_{kopf} °C | Rücklaufverh. g/g | Bezeichnung Fraktion | Massenstrom g/h | FA % | MeOH % | H2O % | TOX % |
| 114 | 97 | 30:1 | Zuspeisung | 600 | 2,2 | 1,7 | 0,5 | 95,6 |
| | | | Sumpf | 566 | 0,06 | <0,01 | 0,07 | 99,9 |
| | | | Destillat | | 9,6 | 11,7 | 3,3 | 75,5 |

Als Sumpfprodukt wird hochreines Trioxan erhalten. Gleichzeitig werden die Leichtersieder mit dem Destillat abgetrennt.

Bedingt durch die überraschend gute Spaltung des Cyclohexyl-Hemiformals im Sumpf von Kolonne 2 läßt sich dort ein gasförmiger Abzug gewinnen, der z.B. in den Verfahrensschritt Formaldehydtrimerisierung rückgeführt werden kann. Weiterhin läßt sich das nahezu formaldehydfreie Sumpfprodukt ebenfalls wiederverwenden. Dies stellt einen besonderen Vorteil des erfindungsgemäßen Verfahrens dar.

Statt der in den Beispielen 1 bis 5 verwendeten, mit strukturierten Packungen bestückten Kolonnen ist bei allen im erfindungsgemäßen Verfahren vorhandenen Destillierapparaten auch der Einsatz anderer Einbauten (wie z.B. Böden oder Füllkörper) denkbar, vorzugsweise jedoch solcher, die eine hohe spezifische Oberfläche aufweisen.

Der am Sumpf von Kolonne 2 abgezogene Strom, enthaltend Cyclohexanol, Trioxan sowie Schwerersieder, wird in Kolonne 8 aufgetrennt. Das Design der Kolonne 8 hängt ganz entscheidend vom Nebenproduktspektrum im Sumpfstrom ab.

### Bezugszeichenliste

- 1: Zuleitung des Ausgangsgemisches
- 2: Kolonne
- 3: Teilkondensator
- 4: Abzug des gasförmigen Gemisches mit Formaldehyd und Trioxan
- 5: Wärmetauscher
- 6: Kolonne
- 7: Verdampfer
- 8: Kolonne
- 9: Kondensator
- 10: Abzug der leichtersiedenden Nebenkomponenten
- 11: Verdampfer
- 12: Abzug des hochreinen Trioxans
- 13: Kondensator
- 14: Abzug der zwischen Trioxan und Alkohol siedenden Nebenkomponenten
- 15: Verdampfer
- 16: Abzug der höhersiedenden Nebenkomponenten
- 17: Abzug des Alkohols
- 18: Teilkondensator

## Patentansprüche

1. Verfahren zur Gewinnung von Trioxan, wobei aus einem flüssigen Gemisch enthaltend Trioxan, Formaldehyd, Alkohol, aus dem Formaldehyd und dem Alkohol gebildete Hemiformale und maximal 5 Gew.-% Wasser das Trioxan durch Destillation abgetrennt und in hoch reiner Form gewonnen wird.

2. Verfahren nach Anspruch 1, **dadurch gekennzeichnet, daß** das flüssige Gemisch einen Gehalt an Trioxan im Bereich von 70 bis 95 Gew.-%, vorzugsweise 83 bis 88 Gew.-% aufweist und das gewonnene hoch reine Trioxan einen Gehalt an Trioxan von mindestens 95 Gew.-%, vorzugsweise mindestens 99 Gew.-% hat.

3. Verfahren nach Anspruch 1 oder 2, **dadurch gekennzeichnet, daß** simultan in separaten Produktströmen der Formaldehyd und der Alkohol gewonnen werden.

4. Verfahren nach einem der Ansprüche 1 bis 3, **dadurch gekennzeichnet, daß** das flüssige Gemisch übliche bei der Herstellung von Trioxan anfallende Nebenkomponenten enthält, die in separaten Produktströmen abgetrennt werden.

5. Verfahren nach einem der Ansprüche 1 bis 4, **dadurch gekennzeichnet, daß** das Trioxan sowie die anderen Produktströme wahlweise flüssig oder gasförmig gewonnen werden.

6. Verfahren nach einem der Ansprüche 1 bis 5, **dadurch gekennzeichnet, daß** die Destillation wahlweise kontinuierlich oder diskontinuierlich bei Unter-, Normal- oder Überdruck in einem Destillierapparat oder in mehreren miteinander verschalteten Destillierapparaten erfolgt.

7. Verfahren nach Anspruch 6, **dadurch gekennzeichnet, daß** die Destillierapparate mehrere Kühl- und Kondensationszonen mit unterschiedlicher Temperatur aufweisen.

8. Verfahren nach Anspruch 1, **dadurch gekennzeichnet, daß** das flüssige Gemisch (1) in einem ersten Destillierapparat (2) in zwei Fraktionen aufgetrennt wird, von denen eine das Trioxan und die leichtersiedenden Komponenten und die andere die schwerersiedenden Komponenten enthält, und die Fraktion mit dem Trioxan und den leichtersiedenden Komponenten in einem zweiten Destillierapparat (6) erneut in zwei Fraktionen aufgetrennt wird, von denen eine (12) Trioxan in hoher Reinheit und die andere (10) die leichtersiedenden Komponenten enthält.

9. Verfahren nach Anspruch 8, **dadurch gekennzeichnet, daß** in dem ersten Destillierapparat (2) eine weitere Fraktion anfällt, welche gasförmig ist und im wesentlichen Formaldehyd enthält.

10. Verfahren nach Anspruch 8 oder 9, **dadurch gekennzeichnet, daß** die im ersten Destillierapparat (2) anfallende Fraktion mit den schwerersiedenden Komponenten in einem weiteren Destillierapparat (8) in zwei oder mehr Fraktionen aufgetrennt wird, wobei eine Fraktion den Alkohol bzw. das Hemiformal in verwertbarer Form enthält und die anderen Fraktionen die Nebenkomponenten enthalten.

11. Verwendung eines nach einem der vorgenannten Verfahren gewonnenen Trioxans mit einem Gehalt an Trioxan von mindestens 95 Gew.-% zur Herstellung von Polymeren und Brennstoffen oder zur Gewinnung von Formaldehyd durch Depolymerisation.

## Claims

1. A process for producing trioxane, the trioxane being separated off from a liquid mixture comprising trioxane, formaldehyde, alcohol, hemiformals formed from the formaldehyde and the alcohol and a maximum of 5% by weight of water by distillation and produced in highly pure form.

2. The process as claimed in claim 1, wherein the liquid mixture has a trioxane content in the range from 70 to 95% by weight, preferably from 83 to 88% by weight, and the highly pure trioxane produced has a trioxane content of at least 95% by weight, preferably at least 99% by weight.

3. The process as claimed in claim 1 or 2, wherein the formaldehyde and the alcohol are produced simultaneously in separate product streams.

4. The process as claimed in one of claims 1 to 3, wherein the liquid mixture comprises usual minor components which arise in the preparation of trioxane and are separated off in separate product streams.

5. The process as claimed in one of claims 1 to 4, wherein the trioxane and the other product streams are optionally produced in the liquid or gaseous state.

6. The process as claimed in one of claims 1 to 5, wherein the distillation is optionally carried out continuously or batchwise at reduced pressure, atmospheric pressure or superatmospheric pressure in a distillation apparatus or in a plurality of distillation apparatuses connected to one another.

7. The process as claimed in claim 6, wherein the distillation apparatuses have a plurality of cooling and condensation zones of differing temperature.

8. The process as claimed in claim 1, wherein the liquid mixture (1) is fractionated in a first distillation apparatus (2) into two fractions, of which one comprises the trioxane and the lower-boiling components and the other comprises the higher-boiling components, and the fraction comprising the trioxane and the lower-boiling components is again fractionated in a second distillation apparatus (6) into two fractions, of which one (12) comprises trioxane at high purity and the other (10) comprises the lower-boiling components.

9. The process as claimed in claim 8, wherein, in the first distillation apparatus (2), a further fraction arises which is gaseous and . essentially comprises formaldehyde.

10. The process as claimed in claim 8 or 9, wherein the fraction comprising the higher-boiling components which is produced in the first distillation apparatus (2) is fractionated in a further distillation apparatus (8) into two or more fractions, one fraction comprising the alcohol or the hemiformal in utilizable form, and the other fractions comprising the minor components.

11. The use of a trioxane produced by one of the abovementioned processes having a trioxane content of at least 95% by weight for preparing polymers and fuels or for producing formaldehyde by depolymerization.

## Revendications

1. Procédé pour l'obtention de trioxanne, dans lequel le trioxanne est séparé par distillation à partir d'un mélange liquide contenant du trioxanne, du formaldéhyde, un alcool, des hémiformals formés à partir du formaldéhyde et de l'alcool et au maximum 5 % en poids d'eau, et obtenu sous forme très pure.

2. Procédé selon la revendication 1, **caractérisé en ce que** le mélange liquide présente une teneur en trioxanne dans la plage allant de 70 à 95 % en poids, de préférence de 83 à 88 % en poids, et le trioxanne très pur obtenu a une teneur en trioxanne d'au moins 95 % en poids, de préférence d'au moins 99 % en poids.

3. Procédé selon la revendication 1 ou 2, **caractérisé en ce que** le formaldéhyde et l'alcool sont obtenus simultanément dans des courants de produits distincts.

4. Procédé selon l'une quelconque des revendications 1 à 3, **caractérisé en ce que** le mélange liquide contient des composants secondaires usuels dans la préparation du trioxanne, qui sont séparés dans des courants de produits distincts.

5. Procédé selon l'une quelconque des revendications 1 à 4, **caractérisé en ce que** le trioxanne ainsi que les autres courants de produits sont obtenus au choix à l'état liquide ou gazeux.

6. Procédé selon l'une quelconque des revendications 1 à 5, **caractérisé en ce que** la distillation s'effectue au choix en continu ou en mode discontinu, sous pression réduite, sous la pression normale ou sous une pression élevée, dans un appareil de distillation ou dans plusieurs appareils de distillation raccordés les uns aux autres.

7. Procédé selon la revendication 6, **caractérisé en ce que** les appareils de distillation comportent plusieurs zones de refroidissement et de condensation à températures différentes.

8. Procédé selon la revendication 1, **caractérisé en ce que**, dans un premier appareil de distillation (2) le mélange liquide (1) est scindé en deux fractions dont l'une contient le trioxanne et les composants à bas point d'ébullition et l'autre contient les composants à haut point d'ébullition, et la fraction contenant le trioxanne et les composants à bas point d'ébullition est scindée à son tour, dans un deuxième appareil de distillation (6), en deux fractions dont l'une (12) contient du trioxanne de grande pureté et l'autre (10) contient les composants à bas point d'ébullition.

9. Procédé selon la revendication 8, **caractérisé en ce que** dans le premier appareil de distillation (2) est formée une autre fraction qui est gazeuse et contient essentiellement du formaldéhyde.

10. Procédé selon la revendication 8 ou 9, **caractérisé en ce que** la fraction formée dans le premier appareil de distillation (2), comportant les composants à haut point d'ébullition, est scindée dans un autre appareil de distillation (8) en deux fractions ou plus, une fraction contenant l'alcool ou l'hémiformal sous forme utilisable, et les autres fractions contenant les composants secondaires.

11. Utilisation d'un trioxanne obtenu selon l'un des procédés précités, ayant une teneur en trioxanne d'au moins 95 % en poids, pour la préparation de polymères et de combustibles ou pour l'obtention de formaldéhyde par dépolymérisation.
